(19) Europäisches Patentamt
European Patent Office
Office européen des brevets

(11) Veröffentlichungsnummer: **0 021 039**
**B1**

(12) # EUROPÄISCHE PATENTSCHRIFT

(45) Veröffentlichungstag der Patentschrift: **09.02.83**

(51) Int. Cl.³: **C 07 C 119/08**

(21) Anmeldenummer: **80102810.1**

(22) Anmeldetag: **21.05.80**

(54) **Verfahren zur Herstellung von aromatischen Azomethinen.**

(30) Priorität: **22.06.79 DE 2925263**

(43) Veröffentlichungstag der Anmeldung: **07.01.81 Patentblatt 81/1**

(45) Bekanntmachung des Hinweises auf die Patenterteilung: **09.02.83 Patentblatt 83/6**

(84) Benannte Vertragsstaaten: **BE CH DE FR GB IT LI NL**

(56) Entgegenhaltungen:
**DE-B-1 793 811**
**HOUBEN-WEYL »Methoden der Organischen Chemie«, 4. Auflage, Band XI/2 1958, GEORG THIEME VERLAG, Stuttgart Seite 77, letzte Zeile bis Seite 78, erste Zeile**

(73) Patentinhaber: **BASF Aktiengesellschaft, Carl-Bosch-Strasse 38, D-6700 Ludwigshafen (DE)**

(72) Erfinder: **Richarz, Winfried, Dr., Königsbergerstrasse 5, D-6081 Stockstadt (DE)**
Erfinder: **Mangold, Dietrich, Dr., Hermann-Walker-Strasse 49, D-6903 Neckargemuend (DE)**
Erfinder: **Girgensohn, Bjoern, Dr., Neckarpromenade 38, D-6800 Mannheim 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



BUNDESDRUCKEREI BERLIN

Verfahren zur Herstellung von aromatischen Azomethinen

Die Erfindung betrifft ein Verfahren zur Herstellung von aromatischen Azomethinen durch Umsetzung von Anilinen mit Formaldehyd und anschließender destillativer Abtrennung der Hauptmenge an Wasser, wobei niedrigsiedende Alkohole in bestimmten Mengen während der Destillation und gewünschtenfalls während der Reaktion oder eines Teiles der Reaktion anwesend sind.

Es ist aus der deutschen Auslegeschrift 1 793 811 bekannt, daß man aromatische Azomethine durch Umsetzung von Anilinen mit Formaldehyd herstellen kann. Das freiwerdende Wasser wird zur Erzielung einer hohen Ausbeute an Endstoff kontinuierlich während der Reaktion durch azeotrope Schleppmittel aus dem Reaktionsgemisch entfernt. Vorzugsweise aromatische Kohlenwasserstoffe wie Benzol, Toluol und die Xylole, aber auch aliphatische Lösungsmittel wie Heptan oder Cyclohexan finden Verwendung. Bei der Umsetzung wird vorzugsweise ein Überschuß an Formaldehyd verwendet, um Formaldehydverluste zu ersetzen, die bei der azeotropen Entfernung des Reaktionswassers aus dem Reaktionsgemisch auftreten. Nach Beendigung der Reaktion, d. h. sobald keine Wasserbildung mehr zu beobachten ist, wird das Produkt vorzugsweise durch Vakuumdestillation gereinigt. Wie alle Beispiele und die Beschreibung zeigen, erfolgt die Umsetzung in Gegenwart von Basen und mit hohen Formaldehydüberschüssen. Es wird darauf hingewiesen, daß die laufende Abtrennung des Wassers gerade während der Reaktion durchgeführt werden muß; die Umsetzung mit gleichzeitiger azeotroper Destillation oder mit gleichzeitiger Abtrennung des Wassers mittels Wasserabsorptionsmittel wird beschrieben. Bei allen Umsetzungen werden hohe Mengen an Toluol benötigt. Führt man die Umsetzung in großtechnischem Maßstab durch, so sind schon wegen der laufenden, azeotropen Destillation mit Toluol sehr lange Reaktionszeiten und Destillationszeiten notwendig. Der für eien möglichs quantitativen Umsatz notwendige Überschuß an Formaldehyd bildet beim Auskreisen des Reaktionswassers Ablagerungen am Kondensator und verursacht Verstopfungen in den Reaktionskesseln und Leitungen. Um diese Ablagerungen zu vermeiden, muß der Kondensator kontinuierlich mit Wasser gespült werden, das zusammen mit dem azeotrop entfernten Reaktionswasser in einem Wasserabscheider vom Schleppmittel abgetrennt werden muß. Da es in den Reaktionskesseln zu Formaldehydverbackungen kommt, ist es notwendig, das gesamte Reaktionsgemisch während des Auskreiseprozesses umzupumpen. Hinzu kommt, daß das Verfahren nur mit den stabileren, in 2-Stellung und insbesondere in 2,6-Stellung durch sterisch hindernde Gruppen, z. B. tert.-Butylgruppen, substituierten Anilinen durchgeführt werden kann; eine Herstellung der interessanten, in 2,6-Stellung durch unverzweigte und insbesondere niedere Alkylgruppen substituierten Aniline wird nicht erwähnt.

Es wurde nun gefunden, daß man aromatische Azomethine der Formel

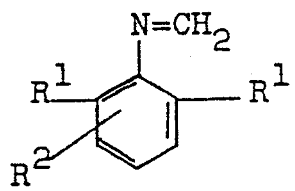

I

worin die einzelnen Reste $R^1$ und $R^2$ gleich oder verschieden sein können und jeweils ein Halogenatom, einen aliphatischen Rest oder eine Alkoxygruppe bedeuten, $R^2$ auch ein Wasserstoffatom bezeichnet, durch Umsetzung von aromatischen Aminen mit Formaldehyd vorteilhaft erhält, wenn man Aniline der Formel

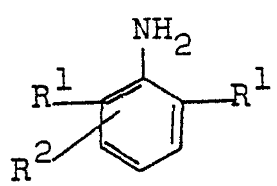

II

worin $R^1$ und $R^2$ die vorgenannte Bedeutung besitzen, mit Formaldehyd umsetzt und dann aus dem Reaktionsgemisch mindestens 98 Gewichtsprozent der gesamten Wassermenge bei einem Druck unterhalb 500 mbar durch Destillation abtrennt, wobei man die Destillation und gewünschtenfalls die Reaktion oder einen Teil der Reaktion in Gegenwart von Alkoholen mit einem Siedepunkt unterhalb 160° C und einer Menge von 0,5 bis 30 Gewichtsprozent, bezogen auf Ausgangsstoff II, durchführt.

Die Umsetzung kann für den Fall der Verwendung von 2,6-Dimethylanilin durch die folgenden Formeln wiedergegeben werden:

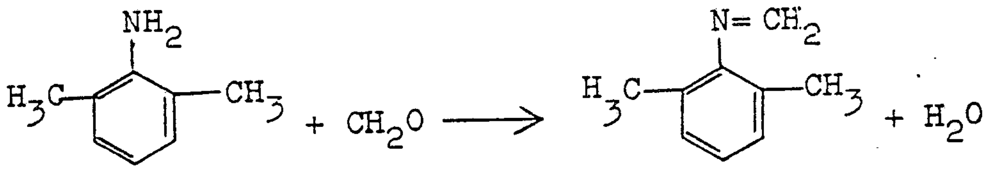

Im Vergleich zu den bekannten Verfahren liefert das Verfahren nach der Erfindung auf einfacherem und wirtschaftlicherem Wege aromatische Azomethine in besserer Raum-Zeit-Ausbeute. Aromatische Kohlenwasserstoffe, insbesondere solche cancerogenen Charakters, als Lösungsmittel werden vermieden.

Das erfindungsgemäße Verfahren wird in der Regel während der Reaktion ohne organische Lösungsmittel durchgeführt; das Verfahren ist umweltfreundlicher und betriebssicherer. Reaktionszeit und Destillationszeit sind wesentlich kürzer. Abscheidung von Formaldehyd in den Anlageteilen und somit Betriebsstörungen bzw. Betriebsstillstand werden vermieden, ohne daß aufwendige Sicherheitsmaßnahmen getroffen werden müssen. Alle diese Vorteile des erfindungsgemäßen Verfahrens sind im Hinblick auf den Stand der Technik überraschend. Gerade auch die interessanten, in 2,6-Stellung durch unverzweigte und insbesondere niedere Alkylgruppen substituierten Aniline können hergestellt werden.

Die Ausgangsstoffe werden miteinander in stöchiometrischer Menge oder im Überschuß, vorzugsweise in einem Verhältnis von 1 bis 3, insbesondere 1,1 bis 1,4 Mol Formaldehyd je Mol Ausgangsstoff II umgesetzt. Der Formaldehyd wird zweckmäßig als Gas oder in Gestalt von unter den Reaktionsbedingungen Formaldehyd bildenden Stoffen wie Paraformaldehyd oder Trioxan, verwendet. Bevorzugte Ausgangsstoffe II und dementsprechend bevorzugte Endstoffe I sind solche, in deren Formeln die einzelnen Reste $R^1$ und $R^2$ gleich oder verschieden sein können und jeweils ein Chloratom, Bromatom, einen Alkylrest, insbesondere einen unverzweigten Alkylrest, oder eine Alkoxygruppe oder einen Perhalogenalkylrest, insbesondere einen Perfluoralkylrest oder Perchloralkylrest, mit jeweils 1 bis 7 Kohlenstoffatomen, oder einen Alkoxyalkylrest mit 2 bis 7 Kohlenstoffatomen bedeuten, $R^2$ auch ein Wasserstoffatom bezeichnet. Die vorgenannten Reste und Ringe können noch durch unter den Reaktionsbedingungen inerte Gruppen, z. B. Alkylgruppen oder Alkoxygruppen mit jeweils 1 bis 4 Kohlenstoffatomen, substituiert sein.

So sind beispielsweise die folgenden Aniline als Ausgangsstoffe II geeignet: 2,6-Dimethyl-, 2,6-Diäthyl-, 2,6-Dipropyl-, 2,6-Diisopropyl-, 2,6-Di-tert.-butyl-, 2,6-Dibutyl-, 2,6-Di-sek.-butyl-, 2,6-Diisobutyl-, 2,6-Dichlor-, 2,6-Dibrom-, 2,6-Dimethoxy-, 2,6-Diäthoxy-, 2,6-Dipropoxy-, 2,6-Diisopropoxy-, 2,6-Dibutoxy-, 2,6-Di-sek.-butoxy-, 2,6-Di-tert.-butoxy-, 2,6-Diisobutoxy-, 2,6-Ditrifluormethyl-, 2,6-Ditrichlormethyl-, 2,6-Diäthoxymethyl-, 2,6-Diäthoxyäthyl-, 2,6-Dimethoxymethyl-, 2,6-Dimethoxyäthyl-, 2-Äthyl-6-methyl-, 2-Methyl-6-chlor-, 2-Methyl-6-brom-, 2-Methyl-6-propyl-, 2-Äthyl-6-propyl-anilin; analoge, zusätzlich in 3-Stellung, 4-Stellung oder 5-Stellung durch die Methyl-, Äthyl-, Propyl-, Isopropyl-, Butyl-, Isobutyl-, sek.-Butyl-, tert.-Butyl-, Methoxy-, Äthoxy-, Propoxy-, Isopropoxy-, Butoxy-, Isobutoxy-, sek.-Butoxy-, tert.-Butoxy-gruppe, ein Chloratom, ein Bromatom, eine Trifluormethylgruppe, eine Trichlormethylgruppe substituierte Aniline.

Die Umsetzung wird im allgemeinen bei einer Temperatur von 60 bis 140, insbesondere von 70 bis 100°C, drucklos oder unter Druck, kontinuierlich oder diskontinuierlich und in der Regel ohne Zusatz organischer Lösungsmittel und ohne Zusatz von Wasser durchgeführt. Das während der Reaktion durch diese Reaktion gebildete Wasser wird im Reaktionsgemisch belassen. Es ist ein überraschendes, wesentliches und vorteilhaftes Merkmal der Erfindung, daß das Reaktionswasser erst dann abdestilliert wird, wenn die Umsetzung im wesentlichen beendet ist.

Im allgemeinen wird man den Alkohol erst nach Beendigung der Umsetzung und vor Beginn der Destillation dem Reaktionsgemisch zusetzen. Gewünschtenfalls kann man den Alkohol aber auch dem Reaktionsgemisch während der Reaktion oder von vornherein dem Ausgangsgemisch zusetzen.

In einer bevorzugten Ausführungsform wird die Reaktion lösungsmittelfrei und ohne Zusatz an Alkohol, lediglich in Anwesenheit des sich bildenden Reaktionswassers, durchgeführt, dann die Gesamtmenge an Alkohol zugegeben und nun die Destillation durchgeführt. Als Alkohole verwendet man zweckmäßig Alkohole mit einem Siedepunkt unterhalb 160°C, vorzugsweise von 65 bis 120°C, insbesondere 65 bis 85°C, vorteilhaft Alkanole und Cycloalkanole, insbesondere Alkanole mit 1 bis 5 Kohlenstoffatomen. Es kommen z. B. als Alkohole in Betracht: Isopropanol, Äthanol, n-Propanol, tert.-Butanol, sek.-Butanol, n-Hexanol, n-Butanol, Isobutanol, Cyclohexanol, n-Pentanol, 2-Pentanol und 2,3-Dimethylpentanol-1. Bevorzugt ist Methanol. Man verwendet Mengen von 0,5 bis 30, zweckmäßig 5 bis 25, vorteilhaft von 10 bis 20 Gewichtsprozent Alkohol, bezogen auf Ausgangsstoff II.

Die Umsetzung kann wie folgt durchgeführt werden: Ein Gemisch von Ausgangsstoff II und Formaldehyd, zweckmäßig Paraformaldehyd, wird während 0,5 bis 3 Stunden bei der Reaktionstemperatur gehalten. Dann setzt man den Alkohol zu und beginnt mit der Destillation des Wassers.

Die Destillation wird bei einem Druck von unterhalb 500 mbar, zweckmäßig von 50 bis 400, insbesondere 80 bis 200 mbar, im allgemeinen bei 60 bis 200, insbesondere 80 bis 160°C, kontinuierlich

oder diskontinuierlich durchgeführt. Man trennt durch die Destillation aus dem Reaktionsgemisch mindestens 98, zweckmäßig von 98 bis 100, insbesondere 99,5 bis 100 Gewichtsprozent der Gesamtmenge an Wasser ab.

Der Rückstand nach der Destillation ist in der Regel zu 90 bis 98 Gewichtsprozent roher Endstoff I. Der reine Endstoff I kann in üblicher Weise, z. B. durch fraktionierte Destillation, isoliert werden. Im allgemeinen wird man aber vorteilhaft den Endstoff sofort weiterverarbeiten; zweckmäßig verdünnt man daher den Destillationsrückstand mit einem für die folgende Synthese geeigneten Lösungsmittel, z. B. Toluol, und führt die Lösung des Endstoffs der Weiterverarbeitung zu. Der nach der Destillation erhaltene Rückstand besteht aus genügend reinem Endstoff I, so daß er für die meisten Folgesynthesen ohne weitere Reinigungsoperationen verwendet werden kann.

Die nach dem Verfahren der Erfindung herstellbaren aromatischen Azomethine I sind wertvolle Ausgangsstoffe für die Herstellung von Herbiziden, Fungiziden, Insektiziden, Bakteriostatika, Fungistatika, insbesondere Vorlauf- und Nachlaufherbizide, Vulkanisationsbeschleuniger. Bezüglich der Verwendung wird auf die vorgenannte Auslegeschrift verwiesen.

Die in den folgenden Beispielen aufgeführten Teile bedeuten Gewichtsteile.

## Beispiel 1

24,2 Teile 2,6-Dimethylanilin und 7,8 Teile Paraformaldehyd werden in einer Rührapparatur vorgelegt und innerhalb 1/2 Stunde auf 80° C erwärmt. Man rührt das Gemisch 30 Minuten bei dieser Temperatur, kühlt auf 60° C ab und setzt 3,2 Teile Methanol zu. Nun wird aus dem Reaktionsgemisch bei einem Druck von 150 mbar ein Gemisch aus Wasser (3,5 Teile), Methanol (3 Teile) und Formaldehyd (1 Teil) abdestilliert. Nach einer Stunde hat die Sumpftemperatur 100° C erreicht. Man hält weitere 15 Minuten bei dieser Temperatur, hebt dann das Vakuum auf und setzt 50 Teile Toluol zum Reaktionsgemisch zu. Man erhält (gaschromatographisch bestimmt) 25,8 Teile (97% der Theorie) N-(2,6-Dimethylphenyl)-N-methylenimin vom Kp 96—104° C (34 mbar).

## Beispiel 2

270 Teile 2-Äthyl-6-methylanilin und 78 Teile Paraformaldehyd werden in einer Rührapparatur 30 Minuten bei 90° C gerührt. Danach kühlt man das Gemisch auf 70° C ab und versetzt es mit 32 Teilen Methanol. Nun destilliert man bei 150 mbar ein Gemisch aus Methanol (31 Teile), Wasser (37 Teile) und Formaldehyd (11 Teile) ab und steigert dabei innerhalb einer Stunde die Sumpftemperatur auf 125° C. Man hält das Gemisch 10 Minuten bei dieser Temperatur und verdünnt danach bei 760 mbar das Gemisch mit 600 Teilen Toluol. Man erhält (gaschromatographisch bestimmt) 289 Teile (95% der Theorie) N-(2-Äthyl-6-methylphenyl)-N-methylenimin vom Kp 78—80° C (5 mbar).

## Beispiel 3

149 Teile 2,6-Diäthylanilin und 39 Teile Paraformaldehyd werden wie in Beispiel 3 beschrieben umgesetzt. Man erhält (gaschromatographisch bestimmt) 154,5 Teile (96% der Theorie) N-(2,6-Diäthylphenyl)-N-methylenimin vom Kp 105—106° C (14 mbar).

**Patentanspruch**

Verfahren zur Herstellung von aromatischen Azomethinen der Formel

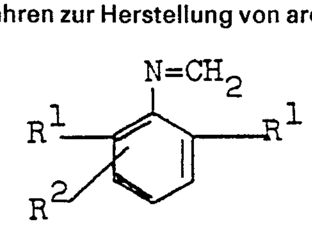

I

worin die einzelnen Reste $R^1$ und $R^2$ gleich oder verschieden sein können und jeweils ein Halogenatom, einen aliphatischen Rest oder eine Alkoxygruppe bedeuten, $R^2$ auch ein Wasserstoffatom bezeichnet, durch Umsetzung von aromatischen Aminen mit Formaldehyd, dadurch gekennzeichnet, daß man Aniline der Formel

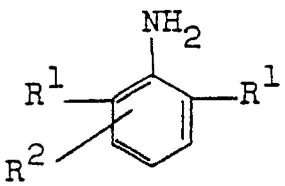

II

worin $R^1$ und $R^2$ die vorgenannte Bedeutung besitzen, mit Formaldehyd umsetzt und dann aus dem Reaktionsgemisch mindestens 98 Gewichtsprozent der gesamten Wassermenge bei einem Druck unterhalb 500 mbar durch Destillation abtrennt, wobei man die Destillation und gewünschtenfalls die Reaktion oder einen Teil der Reaktion in Gegenwart von Alkoholen mit einem Siedepunkt unterhalb 160° C und in einer Menge von 0,5 bis 30 Gewichtsprozent, bezogen auf Ausgangsstoff II, durchführt.

**Claim**

A process for the preparation of aromatic azomethines of the formula

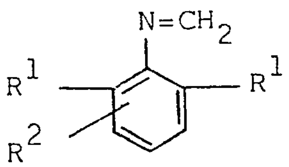

I

where the individual radicals $R^1$ and $R^2$ may be identical or different and each is halogen, an aliphatic radical or alkoxy, and $R^2$ may also be hydrogen, by reacting an aromatic amine with formaldehyde, wherein an aniline of the formula

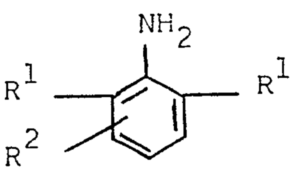

II

where $R^1$ and $R^2$ have the above meanings, is reacted with formaldehyde and thereafter at least 98 percent by weight of the total amount of water is removed from the reaction mixture by distillation under a pressure of less than 500 mbar, the distillation, and, if desired, the reaction or a part thereof, being carried out in the presence of from 0.5 to 30 percent by weight, based on starting material II, of an alcohol having a boiling point below 160° C.

**Revendication**

Procédé de préparation d'azométhines aromatiques de la formule

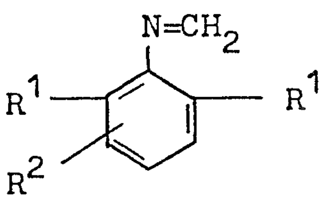

(I)

dans laquelle les divers restes $R^1$ et $R^2$, qui peuvent être identiques ou différents, désignent chacun un atome d'halogène, un reste aliphatique ou un groupe alcoxy, $R^2$ pouvant en outre représenter un atome d'hydrogène par réaction d'amines aromatiques avec le formaldéhyde, caractérisé en ce que l'on fait réagir une aniline de la formule

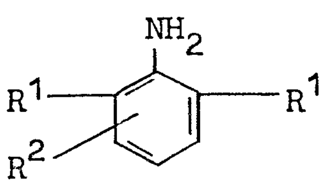

. (II)

dans laquelle $R^1$ et $R^2$ possèdent la signification définie, avec du formaldéhyde, puis on élimine au moins 98% en poids de la quantité totale d'eau présente dans le mélange réactionnel par une distillation sous une pression inférieure à 500 millibars, cette distillation et le cas échéant la réaction, ou une partie de la réaction étant réalisées en présence d'alcools d'un point d'ébullition inférieur à 160° C en une proportion de 0,5 à 30% en poids par rapport au composé de départ II.